# EUROPÄISCHE PATENTSCHRIFT

(11) **EP 1 359 213 B1**
(45) Veröffentlichungstag und Bekanntmachung des Hinweises auf die Patenterteilung: **21.03.2007**
(21) Anmeldenummer: 02009724.2
(22) Anmeldetag: 30.04.2002
(51) Int. Cl.: C11D 17/00, C11D 1/83, A61K 8/11, A61K 8/41, A61K 8/44, A61K 8/46, A61K 8/60, A61K 8/81, A61Q 5/02, A61Q 19/10

(54) **Wässrige Tensidzubereitungen**
Aqueous Detergent Compositions
Compositions tensioactives aqueuses

(43) Veröffentlichungstag der Anmeldung: 05.11.2003
(73) Patentinhaber: Cognis IP Management GmbH, 40589 Düsseldorf (DE)
(72) Erfinder: De Moragas, Maria, Dr., 08310 Argentona (Barcelona) (ES); Burgo, Antonino, 22063 Cantu (CO) (IT); Fabry, Bernd, Dr., 41352 Korschenbroich (DE)

(56) Entgegenhaltungen:
- EP-A- 1 253 193
- WO-A-00/32733
- WO-A-00/32735
- WO-A-00/42147
- WO-A-00/52125
- WO-A-99/02634
- DE-A- 19 918 267

## Beschreibung

### Gebiet der Erfindung

Die Erfindung befindet sich auf dem Gebiet der verkapselten Wirkstoffe und betrifft die Verwendung von Tensidzubereitungen zur Herstellung von Haarshampoos.

### Stand der Technik

Die Verkapselung von Wirkstoffen hat sich in den letzten Jahren als interessante Möglichkeit erwiesen, zum einen ansonsten miteinander unverträgliche Stoffe in einer Rezeptur zu formulieren als auch bestimmte Wirkstoffe kontrolliert bzw. zeitverzögert freizusetzen. Mitunter werden Mikrokapseln, die mit Farbstoffen gefüllt sind, Produkten, wie z.B. Geschirrspülmitteln, Shampoos oder Stylingprodukten aus nur aus ästhetischen Mitteln zugesetzt. Viele dieser Zubereitungen enthalten notwendigerweise oberflächenaktive Substanzen, welche jedoch den Nachteil besitzen, dass sie die Hüllmembran der meisten im Markt befindlichen Mikrokapseln mehr oder minder rasch auflösen, was dann zur verfrühten und unerwünschten Freisetzung der darin enthaltenen Wirkstoffe führt. So wird beispielsweise in der internationalen Patentanmeldung WO 00/65020 (Henkel) ein manuelles Geschirrspülmittel beansprucht, welches verkapselte Wirkstoffe in einer tensidischen Phase enthaltend anionische und amphotere Tenside enthält. Obschon die anwendungstechnischen Leistungen dieser Zubereitung durchaus zufriedenstellend sind, weist sie jedoch den oben beschriebenen Nachteil auf, dass die Mikrokapseln, zumal bei höheren Temperaturen, nicht ausreichend beständig sind. Eine wasserklare Zubereitung, welche mit dunkelblauem Farbstoff beladene Mikrokapseln beinhaltet, färbt sich z.B. infolge der fortschreitenden Auflösung der Hüllmembranen innerhalb von 4 Wochen himmelblau. Nach Untersuchungen der Anmelderin kann für diesen Effekt insbesondere die Mitverwendung der amphoteren Tenside verantwortlich gemacht werden.

Die Aufgabe der Erfindung hat folglich darin bestanden, neue aniontensidische Zubereitungen vergleichbarer Art zur Verfügung zu stellen, die bei entsprechendem Leistungsniveau frei von den oben beschriebenen Nachteilen sind, d.h. die Mikrokapseln über einen deutlich längeren Zeitraum stabil halten, ohne die Hüllmembranen aufzulösen.

### Beschreibung der Erfindung

Gegenstand der Erfindung ist die Verwendung von wässrigen Tensidzubereitungen mit erhöhter Viskosität, enthaltend
- (a): anionische Tenside,
- (b1): Alkyl- und/oder Alkenyloligoglykoside und/oder
- (b2): Aminoxide
- (c): Verdickungsmittel,
- (d): verkapselte Wirkstoffe sowie gegebenenfalls
- (e): bis zur Hälfte der Gewichtsmenge an nichtionischen Tensiden (Komponente b1 und b2) amphotere und/oder zwitterionische Tenside
zur Herstellung von Haarshampoos.

Überraschenderweise wurde gefunden, dass der Austausch von amphoteren bzw. zwitterionischen gegen nichtionische Tenside vom Typ der Alkyl- und/oder Alkenyloligoglykoside und/oder Aminoxide zu einer deutlich verbesserten Beständigkeit der Mikrokapseln führt, d.h. die Mikrokapseln widerstehen der tensidischen Umgebung längere Zeit ohne sich aufzulösen und damit die Inhaltsstoffe vorzeitig freizusetzen. Die Erfindung schließt die Erkenntnis ein, dass der Einsatz der nichtionischen Tenside sogar die Mitverwendung von Betainen bis etwa zur Hälfte der Gewichtsmenge der nichtionischen Tenside zulässt.

### Anionische Tenside

Typische Beispiele für anionische Tenside, die die Komponente (a) darstellen, sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Besonders bevorzugt ist der Einsatz von anionischen Tensiden vom Typ der Alkansulfonate, Alkylsulfate, Acylglutamate sowie insbesondere der Alkylethersulfate.

Alkylethersulfate stellen anionische Tenside dar, die durch Sulfatierung und nachfolgende Neutralisation von Polyethylenglycolethern erhalten werden. Sie folgen vorzugsweise der Formel **(I),**

**R¹O(CH₂CH₂O)ₙSO₃X** (I)

in der R¹ für einen linearen oder verzweigten Alkyl- oder Alkenylrest mit 6 bis 22 und vorzugsweise 12 bis 18 Kohlenstoffatomen , n für Zahlen von 1 bis 10, vorzugsweise 2 bis 5 und X für Alkali, Erdalkali, Ammonium, Alkylammonium, Alkanolammonium oder Glucammonium steht. Typische Beispiele sind die Sulfate der Anlagerungsprodukte von 1 bis 10, vorzugsweise 2 bis 5 Mol Ethylenoxid an Capronalkohol, Caprylalkohol, Caprinalkohol, Laurylalkohol, Myristylalkohol, Cetylalkohol, Stearylalkohol, Isostearylalkohol, Oleylalkohol, Cocoylalkohol, Cetearylalkohol, Behenylalkohol, Erucylalkohol sowie deren Gemische in Form der entsprechenden Natrium-, Kalium-, Ammonium- oder Triethanolammoniumsalze. besonders bevorzugt ist der Einsatz von Natriumlaurylethersulfat (Sodium Laureth Sulfate).

### Alkyl-und/oder Alkenyloligoelykoside

Alkyl- und Alkenyloligoglykoside, die die Komponente (b1) bilden, stellen bekannte nichtionische Tenside dar, die der Formel **(II)** folgen,

**R²O-[G]ₚ** (II)

in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Aminoxide

Aminoxide, die die Komponente (b2) bilden, stellen oberflächenaktive Stoffe dar, die üblicherweise zu den nichtionischen Tensiden gezählt werden. Sie werden beispielsweise durch Oxidation tertiärer Amine mit Peroxiden erhalten und folgen vorzugsweise der Formel (III),

**R³R⁴R⁵N->O** **(III)**

in der R³ für einen linearen oder verzweigten Alkyl- oder Alkenylrest mit 6 bis 22, vorzugsweise 12 bis 18 Kohlenstoffatomen oder einen Benzylrest und R⁴ und R⁵ unabhängig voneinander für R³ oder lineare oder verzweigte Alkylreste mit 1 bis 4 Kohlenstoffatomen steht. Typische Beispiele sind die Oxide von Dimethylbenzylamin, Dimethyllaurylamin, Dimethylmyristylamin, Dimethylcetylamin, Dimethylstearylamin, Dimethylisostearylamin, Dimethyloleylamin, Dimethylcocoylamin, Dimethylcetearylamin, Dimethylbehenylamin, Dimethylerucylamin, Diethyllaurylamin, Diethylmyristylamin, Diethylcetylamin, Diethylstearylamin, Diethylisostearylamin, Diethyloleylamin, Diethylcocoylamin, Diethylcetearylamin, Diethylbehenylamin, Diethylerucylamin, Methyldilaurylamin, Methyldimyristylamin, Methyldicetylamin, Methyldistearylamin, Methyldiisostearylamin, Methyldioleylamin, Methyldicocoylamin, Methyldicetearylamin, Methyldibehenylamin, Methyldierucylamin, Trilaurylamin, Tricocoylamin und Tricetearylamin.

### Verdickungsmittel

Die Verdickungsmittel (Komponente c) besitzen die Aufgabe, den Zubereitungen eine solch hohe Viskosität zu verleihen, dass die Mikrokapseln stabil dispergiert bleiben, d.h. nicht im Laufe der Lagerung zu boden sinken. Unter dem Begriff erhöhter Viskosität ist somit eine solche Rheologie zu verstehen, die die Stabilisierung der Mikrokapseln sicherstellt. Üblicherweise liegen derartige Viskositäten (bestimmt nach Brookfield, RVT-Viskosimeter, 20 °C, Spindel 1, 10 Upm) oberhalb von 100 und vorzugsweise oberhalb von 500 mPas, vorzugsweise im Bereich von 200 bis 2.000 und insbesondere 500 bis 1.000 mPas.

Geeignete Verdickungsmittel sind alle die Stoffe, die den Tensidzubereitungen eine entsprechend hohe Viskosität verleihen. Vorzugsweise handelt es sich jedoch um polymere Verbindungen, da diese in der Lage sind, in den wässrigen Zubereitungen ein dreidimensionales Netz aufzubauen, in welchem die Mikrokapseln stabilisiert werden. Typische Beispiele sind Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt.

### Wirkstoffe

Die für die Mikroverkapselung in Betracht kommenden Wirkstoffe (Komponente d) können in zwei Gruppen unterteilt werden, nämlich in solche, die andernfalls mit den Bestandteilen der wässrigen Phase reagieren würden und solchen, die zwar chemisch stabil sind, aber erst gezielt während der Anwendung freigesetzt werden sollen. Zur ersten Gruppe gehören insbesondere kationische Tenside, die mit den anionischen Bestandteilen Salze bilden würden. Zur zweiten Gruppe gehören Stoffe, die beim Inkontaktbringen der Tensidzubereitungen, also beispielsweise beim manuellen Spülvorgang, die Haut schützen und pflegen sollen. Hierzu zählen biogene Wirkstoffe, wie beispielsweise Vitamin E und dessen Derivate (z.B. Tocopherol, Tocopherolacetat, Tocopherolpalmitat), Vitamin A und dessen Derivate (z.B. Carotine), Koffein, Ascorbinsäure, (Desoxy)Ribonukleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, Chitosan, Menthol, Squalan, essentielle Öle (z.B. Jojobaöl), pflanzliche Proteine und deren Hydrolyseprodukte sowie Pflanzenextrakte und Vitaminkomplexe zu verstehen. Besonders bevorzugt ist der Einsatz von Squalan, Chitosan, Menthol, Retinol (Vitamin A), Koffein, pflanzlichen Proteinen und deren Hydrolyseprodukten, Carotinen und Jojobaöl, da diese zum Gleichgewicht der cutanen Hydrolipidschicht beitragen, dem Wasserverlust vorbeugen, und der Haut beispielsweise nach dem Spülen ein weiches und elastisches Gefühl verleihen.

Schließlich kommen als zu verkapselnde Wirkstoffe auch solche Stoffe in Betracht, die keinen direkten Beitrag zur Leistung der Mittel erbringen, sondern diesen aus ästhetischen Gründen zugesetzt werden, wie z.B. Farbstoffe, Farbpigmente, Parfümöle und Aromen. Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

Der Anteil der Wirkstoffe an den Mikrokapseln kann 1 bis 30, vorzugsweise 5 bis 25 und insbesondere 15 bis 20 Gew.-% betragen.

### Mikrokapseln

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammem angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929**]. Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
- (a1): aus Gelbildnern, Chitosanen und Wirkstoffen eine Matrix zubereitet,
- (a2): gegebenenfalls die Matrix in einer Ölphase dispergiert,
- (a3): die dispergierte Matrix mit wässrigen Lösungen anionischer Polymere behandelt und gegebenenfalls dabei die Ölphase entfernt.
oder
- (b1): aus Gelbildnern, anionischen Polymeren und Wirkstoffen eine Matrix zubereitet,
- (b2): gegebenenfalls die Matrix in einer Ölphase dispergiert,
- (b3): die dispergierte Matrix mit wässrigen Chitosanlösungen behandelt und gegebenenfalls dabei die Ölphase entfernt.
oder
- (c1): wässrige Wirkstoffzubereitungen mit Ölkörpern in Gegenwart von Emulgatoren zu O/W-Emulsionen verarbeitet,
- (c2): die so erhaltenen Emulsionen mit wässrigen Lösungen anionischer Polymere behandelt,
- (c3): die so erhaltene Matrix mit wässrigen Chitosanlösungen in Kontakt bringt und
- (c4): die so erhaltenen Verkapselungsprodukte von der wässrigen Phase abtrennt.

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

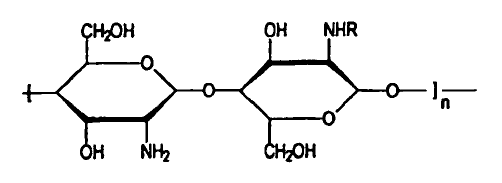

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffaungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

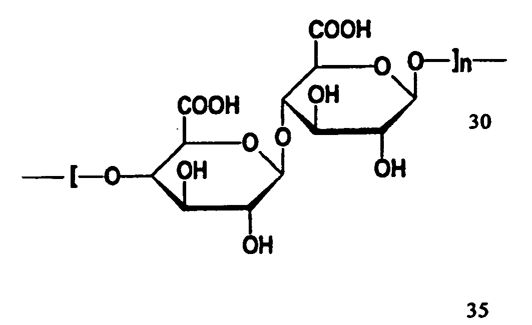

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

### • Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### • Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### • Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### • Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### • Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### • Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### • Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Chitosan und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Chitosanen durchführen.

In einem alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

### Amphotere bzw. zwitterionische Tenside

Wie schon eingangs erläutert, wurde überraschenderweise gefunden, dass die nichtionischen Tenside, die die Komponenten (b1) und (b2) bilden, nicht nur die Beständigkeit der Mikrokapseln in der wässrigen Zubereitung substantiell verbessern, sie erlauben sogar die partielle Mitverwendung der ansonsten wegen ihrer destabilisierenden Effekte eher unerwünschten amphoteren und zwitterionischen Tenside. Diese können - mit geringen Einbußen bei der Stabilisierung - in den Formulierungen bis etwa zur Hälfte, vorzugsweise bis zu 40 % der Menge an nichtionischen Tensiden (b1+b2) geduldet werden; darüber hinaus nimmt die Beständigkeit der Kapseln wieder stark ab.

Beispiele für geeignete amphotere bzw. zwitterionische Tenside (Komponente e) sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar, die der Formel **(IV)** folgen, 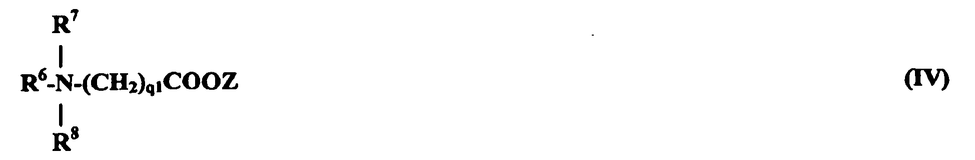 in der R⁶ für Alkyl- und/oder Alkenylreste mit 6 bis 22 Kohlenstoffatomen, R⁷ für Wasserstoff oder Alkylreste mit 1 bis 4 Kohlenstoffatomen, R⁸ für Alkylreste mit 1 bis 4 Kohlenstoffatomen, q1 für Zahlen von 1 bis 6 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethyl-amin, O-leyldimethylamin, C_{16/18}-Talgalkyldimethylamin sowie deren technische Gemische.

Weiterhin kommen auch Carboxyalkylierungsprodukte von Amidoaminen in Betracht, die der Formel (**V**) folgen, 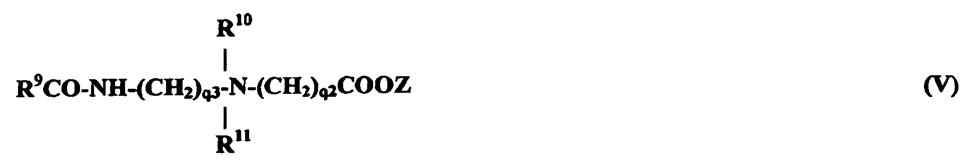 in der R⁹CO für einen aliphatischen Acylrest mit 6 bis 22 Kohlenstoffatomen und 0 oder 1 bis 3 Doppelbindungen, R¹⁰ für Wasserstoff oder Alkylreste mit 1 bis 4 Kohlenstoffatomen, R¹¹ für Alkylreste mit 1 bis 4 Kohlenstoffatomen, q2 für Zahlen von 1 bis 6, q3 für Zahlen von 1 bis 3 und Z wieder für ein Alkali- und/oder Erdalkalimetall oder Ammonium steht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethyl-aminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Tensidzubereitungen

In einer bevorzugten Ausführungsform der vorliegenden Erfindung werden solche Tensidzubereitungen eingesetzt, die
- (a): 1 bis 40, vorzugsweise 5 bis 30 und insbesondere 10 bis 20 Gew.-% anionische Tenside,
- (b1): 1 bis 15, vorzugsweise 2 bis 10 und insbesondere 4 bis 8 Gew.-% Alkyl- und/oder Alkenyloligoglykoside und/oder
- (b2): 1 bis 15, vorzugsweise 2 bis 10 und insbesondere 4 bis 8 Gew.-% Aminoxide,
- (c): 0,1 bis 5, vorzugsweise 0,5 bis 2 und insbesondere 1 bis 1,5 Gew.-% Verdickungsmittel,
- (d): 0,1 bis 5, vorzugsweise 0,5 bis 2 und insbesondere 1 bis 1,5 Gew.-% verkapselte Wirkstoffes sowie gegebenenfalls
- (e): 0 bis 7, vorzugsweise 1 bis 5 und insbesondere 2 bis 4 Gew.-% amphotere und/oder zwitterionische Tenside
mit der Maßgabe enthalten, dass sich die Mengenangaben mit Wasser und gegebenenfalls weiteren Hilfs- und Zusatzstoffen zu 100 Gew.-% ergänzen.

### Gewerbliche Anwendbarkeit

Die erfindungsgemäß verwendeten Zubereitungen besitzen eine ausgezeichnete Detergensleistung bei optimaler Hautverträglichkeit. Im einfachsten Fall werden die Mittel direkt für die gewünschte Anwendung eingesetzt, andernfalls werden sie mit Wasser auf die gewünschte Anwendungskonzentration verdünnt oder dienen als Compounds für die Herstellung der Endprodukte.

### Beispiele

Die folgenden Tensidzubereitungen wurden bei 25 °C über einen Zeitraum von 6 Wochen gelagert. Die enthaltenen Mikrokapseln vom Typ Primaspheres® (Cognis) waren mit 10 Gew.-% dunkelblauem Farbstoff beladen. Die Beständigkeit der Mikrokapseln in der tensidischen Umgebung wurde optisch über die Freisetzung des Farbstoffs aus den Kapseln in die umgebende Phase verfolgt. Die Ergebnisse sind in Tabelle 1 zusammengefasst. Die Beispiele 1 bis 3 sind erfindungsgemäß, Beispiel V1dient zum Vergleich.

**Tabelle 1**

| **Freisetzung von Farbstoffen aus Mikrokapseln als Funktion der Zeit (Mengenangaben als Gew.-%)** | | | | |
|---|---|---|---|---|
| **Zusammensetzung** | **1** | **2** | **3** | **V1** |
| Sodium Laurethsulfate | 20,0 | 20,0 | 20,0 | 20,0 |
| Coco Glucosides | 10,0 | - | 6,0 | - |
| Benzyldimethylaminoxid | - | 10,0 | - | - |
| Cocamidopropylbetain | - | - | 4,0 | 10,0 |
| Carbopol | 0,5 | 0,5 | 0,5 | 0,5 |
| Mikrokapseln | 1,0 | 1,0 | 1,0 | 1,0 |
| Wasser | ad 100 | | | |

| ***Färbung der wässrigen Phase*** | | | | |
|---|---|---|---|---|
| - nach 1 w | farblos | farblos | farblos | farblos |
| - nach 2 w | farblos | farblos | farblos | farblos |
| - nach 3 w | farblos | farblos | farblos | farblos |
| - nach 4 w | farblos | farblos | farblos | leicht blau |
| - nach 5 w | farblos | farblos | leicht blau | blau |
| - nach 6 w | farblos | farblos | leicht blau | blau |

Man erkennt, dass der Austausch der amphoteren gegen die speziellen nichtionischen Tenside zu einer Stabilisierung der Mikrokapseln gegenüber Auflösungserscheinungen führt. Selbst ein Teilaustausch führt bereits gegenüber der Vergleichsformulierung zu einer deutlichen Verbesserung.

## Patentansprüche

1. Verwendung von wässrigen Tensidzubereitungen mit erhöhter Viskosität, enthaltend
(a) anionische Tenside,
(b1) Alkyl- und/oder Alkenyloligoglykoside und/oder
(b2) Aminoxide
(c) Verdickungsmittel,
(d) verkapselte Wirkstoffe sowie gegebenenfalls
(e) bis zur Hälfte der Gewichtsmenge an nichtionischen Tensiden (Komponente und b2) amphotere und/oder zwitterionische Tenside
zur Herstellung von Haarshampoos.

2. Verwendung nach Anspruch 1, **dadurch gekennzeichnet, dass** man anionische Tenside einsetzt, die ausgewählt sind aus der Gruppe, die gebildet wird von Seifen, Alkylbenzolsulfonaten, Alkansulfonaten Olefinsulfonaten, Alkylethersulfonaten, Glycerinethersulfonaten, a-Methylestersulfonaten, Sulfofettsäuren, Alkylsulfaten, Alkylethersulfaten, Glycerinethersulfaten, Fettsäureethersulfaten, Hydroxymischethersulfaten, Monoglycerid-(ether)sulfaten, Fettsäureamid(ether)sulfaten, Mono- und Dialkylsulfosuccinaten, Mono- und Dialkylsulfosuccinamaten Sulfotriglyceriden, Amidseifen, Ethercarbonsäuren und deren Salzen Fettsäureisethionaten Fettsäuresarcosinaten, Fettsäuretauriden, N-Acylaminosäuren, Alkyloligoglucosidsulfaten, Proteinfettsäurekondensaten und Alkyl(ether)phosphaten.

3. Verwendung nach Anspruch 2, **dadurch gekennzeichnet, dass** man als anionische Tenside Alkansulfonate, Alkylsulfate, Alkylethersulfate und/oder Acylglutamate einsetzt

4. Verwendung nach mindestens einem der Ansprüche 1 bis 3, **dadurch gekennzeichnet, dass** man als anionische Tenside Alkylethersulfate der Formel **(I)** einsetzt,
**R¹O(CH₂CH₂O)ₙSO₃X** (I)
in der R¹ für einen linearen oder verzweigten Alkyl- oder Alkenylrest mit 6 bis 22 Kohlenstoffatomen , n für Zahlen von 1 bis 10 und X für Alkali, Erdalkali, Ammonium, Alkylammonium, Alkanolammonium oder Glucammonium steht.

5. Verwendung nach mindestens einem der Ansprüche 1 bis 4, **dadurch gekennzeichnet, dass** man Alkyl- und Alkenyloligoglykoside der Formel **(II)** einsetzt,
**R²O-[G]ₚ** (II)
in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

6. Verwendung nach mindestens einem der Ansprüche 1 bis 5, **dadurch gekennzeichnet, dass** sie Aminoxide der Formel **(III)** einsetzt,
**R³R⁴R⁵-N->O** (III)
in der R³ für einen linearen oder verzweigten Alkyl- oder Alkenylrest mit 6 bis 22, vorzugsweise 12 bis 18 Kohlenstoffatomen oder einen Benzylrest und R⁴ und R⁵ unabhängig voneinander für R³ oder lineare oder verzweigte Alkylreste mit 1 bis 4 Kohlenstoffatomen steht.

7. Verwendung nach mindestens einem der Ansprüche 1 bis 6, **dadurch gekennzeichnet, dass** man Verdickungsmittel einsetzt, die ausgewählt sind aus der Gruppe, die gebildet wird von hydrophilen Kieselsäuren, Polysacchariden, Carboxymethylcellulosen, Hydroxyethyl- und Hydroxypropylcellulosen, höhermolekularen Polyethylenglycolmono- und -diestern von Fettsäuren, Polyacrylaten, Polyacrylamiden, Polyvinylalkoholen, Polyvinylpyrrolidonen sowie Bentoniten.

8. Verwendung nach mindestens einem der Ansprüche 1 bis 7, **dadurch gekennzeichnet, dass** man mikroverkapselte Wirkstoffe einsetzt, die ausgewählt sind aus der Gruppe, die gebildet wird von kationischen Tensiden, biogenen Wirkstoffen, Farbstoffen, Farbpigmenten, Parfümölen, Aromen und deren Gemischen.

9. Verwendung nach Anspruch 8, **dadurch gekennzeichnet, dass** man biogene Wirkstoffe einsetzt, die ausgewählt sind aus der Gruppe, die gebildet wird von Squalan, Chitosan, Menthol, Retinol (Vitamin A), Koffein, pflanzlichen Proteinen und deren Hydrolyseprodukten, Carotinen und Jojobaöl sowie deren Gemischen.

10. Verwendung nach mindestens einem der Ansprüche 1 bis 9, **dadurch gekennzeichnet, dass** man Mikrokapseln mit mittleren Durchmessern im Bereich von 0,0001 bis 5 mm, bestehend aus einer Hüllmembran und einer die Wirkstoffe enthaltenden Matrix, einsetzt, die **dadurch** erhältlich sind, dass man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.
oder
(c1) wässrige Wirkstoffzubereitungen mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

11. Verwendung nach mindestens einem der Ansprüche 1 bis 10, **dadurch gekennzeichnet, dass** man als weitere Komponente (e) Alkylbetaine der Formel (IV) einsetzt, 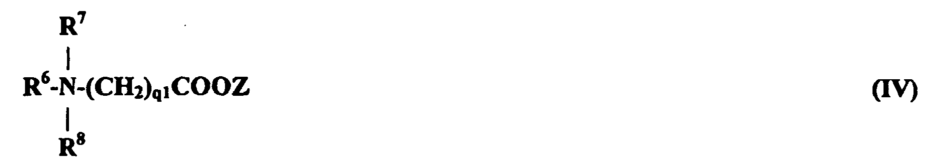 in der R⁶ für Alkyl- und/oder Alkenylreste mit 6 bis 22 Kohlenstoffatomen, R⁷ für Wasserstoff oder Alkylreste mit 1 bis 4 Kohlenstoffatomen, R⁸ für Alkylreste mit 1 bis 4 Kohlenstoffatomen, q1 für Zahlen von 1 bis 6 und Z für ein Alkali- und/oder Erdalkalimetall oder Ammonium steht.

12. Verwendung nach mindestens einem der Ansprüche 1 bis 11, **dadurch gekennzeichnet, dass** man als weitere Komponente (e) Alkylamidobetaine der Formel (V) einsetzt, 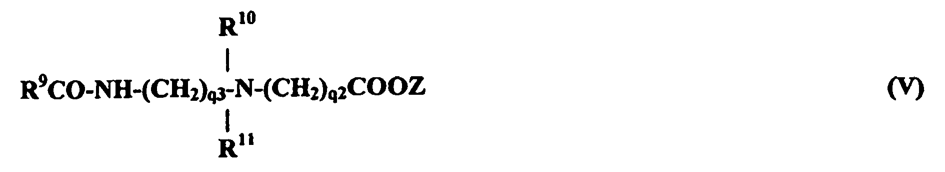 in der R⁹CO für einen aliphatischen Acylrest mit 6 bis 22 Kohlenstoffatomen und 0 oder 1 bis 3 Doppelbindungen, R¹⁰ für Wasserstoff oder Alkylreste mit 1 bis 4 Kohlenstoff atomen, R¹¹ für Alkylreste mit 1 bis 4 Kohlenstoffatomen, q2 für Zahlen von 1 bis 6, q3 für Zahlen von 1 bis 3 und Z für ein Alkali- und/oder Erdalkalimetall oder Ammonium steht.

13. Verwendung nach mindestens einem der Ansprüche 1 bis 12, **dadurch gekennzeichnet, dass** man
(a) 1 bis 40 Gew.-% anionische Tenside,
(b1) 1 bis 15 Gew.-% Alkyl- und/oder Alkenyloligoglykoside und/oder
(b2) 1 bis 15 Gew.-% Aminoxide,
(c) 0,1 bis 5 Gew.-% Verdickungsmittel,
(d) 0,1 bis 5 Gew.-% verkapselte Wirkstoffes sowie gegebenenfalls
(e) 0 bis 7 Gew.-% amphotere und/oder zwitterionische Tenside
mit der Maßgabe einsetzt, dass sich die Mengenangaben mit Wasser und gegebenenfalls weiteren Hilfs- und Zusatzstoffen zu 100 Gew.-% ergänzen.

## Claims

1. Use of high-viscosity aqueous surfactant preparations containing
(a) anionic surfactants,
(b1) alkyl and/or alkenyl oligoglycosides and/or
(b2) amine oxides.
(c) thickeners,
(d) encapsulated active components and optionally
(e) up to half the quantity by weight of nonionic surfactants (component b1 and b2), amphoteric and/or zwitterionic surfactants
for the production of hair shampoos.

2. Use claimed in claim 1, **characterized in that** anionic surfactants selected from the group consisting of soaps, alkyl benzenesulfonates, alkanesulfonates, olefin sulfonates, alkylether sulfonates, glycerol ether sulfonates, α-methyl ester sulfonates, sulfofatty acids, alkyl sulfates, alkyl ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, alkyl oligoglucoside sulfates, protein fatty acid condensates and alkyl (ether) phosphates are used.

3. Use claimed in claim 2, **characterized in that** alkanesulfonates, alkyl sulfates, alkyl ether sulfates and/or acyl glutamates are used as the anionic surfactants.

4. Use claimed in at least one of claims 1 to 3, **characterized in that** alkyl ether sulfates corresponding to formula **(I)**:
**R¹O(CH₂CH₂O)ₙSO₃X** (I)
in which R¹ is a linear or branched alkyl or alkenyl group containing 6 to 22 carbon atoms, n is an integer of 1 to 10 an X represents alkali metal, alkaline earth metal, ammonium, alkylammonium, alkanolammonium or glucammonium,
are used as the anionic surfactants.

5. Use claimed in at least one of claims 1 to 4, **characterized in that** alkyl and alkenyl oligoglycosides corresponding to formula **(II):**
**R²O-[G]ₚ** (II)
in which R² is an alkyl and/or alkenyl group containing 4 to 22 carbon atoms, G is a sugar residue containing 5 or 6 carbon atoms and p is a number of 1 to 10, are used.

6. Use claimed in at least one of claims 1 to 5, **characterized in that** amino oxides corresponding to formula **(III):**
**R³R⁴R⁵-N->O** (III)
in which R³ is a linear or branched alkyl or alkenyl group containing 6 to 22 and preferably 12 to 18 carbon atoms or a benzyl group and R⁴ and R⁵ independently of one another have the same meaning as R³ or represent linear or branched alkyl groups containing 1 to 4 carbon atoms, are used.

7. Use claimed in at least one of claims 1 to 6, **characterized in that** thickeners selected from the group consisting of hydrophilic silicas, polysaccharides, carboxymethyl celluloses, hydroxyethyl and hydroxypropyl celluloses, relatively high molecular weight polyethylene glycol mono- and diesters of fatty acids, polyacrylates, polyacrylamides, polyvinyl alcohols, polyvinyl pyrrolidones and bentonites are used.

8. Use claimed in at least one of claims 1 to 7, **characterized in that** microencapsulated active components selected from the group consisting of cationic surfactants, biogenic agents, dyes, pigments, perfume oils, flavours and mixtures thereof are used.

9. Use claimed in claim 8, **characterized in that** biogenic agents selected from the group consisting of squalane, chitosan, menthol, retinol (vitamin A), caffeine, vegetable proteins and hydrolysis products thereof, carotenes and jojoba oil and mixtures thereof are used.

10. Use claimed in at least one of claims 1 to 9, **characterized in that** microcapsules with mean diameters of 0.0001 to 5 mm consisting of a membrane and a matrix containing the active components which are obtainable by
(a1) preparing a matrix from gel formers, chitosans and active components,
(a2) optionally dispersing the matrix in an oil phase,
(a3) treating the dispersed matrix with aqueous solutions of anionic polymers and optionally removing the oil phase
or
(b1) preparing a matrix from gel formers, anionic polymers and active components,
(b2) optionally dispersing the matrix in an oil phase,
(b3) treating the dispersed matrix with aqueous chitosan solutions and optionally removing the oil phase
or
(c1) processing aqueous active component preparations with oil components in the presence of emulsifiers to form o/w emulsions,
(c2) treating the emulsions thus obtained with aqueous solutions of anionic polymers,
(c3) contacting the matrix thus obtained with aqueous chitosan solutions and
(c4) separating the encapsulation products thus obtained from the aqueous phase.

11. Use claimed in at least one of claims 1 to 10, **characterized in that** alkyl betaines corresponding to formula **(IV):** 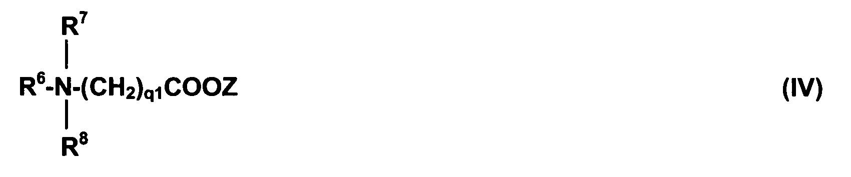 in which R⁶ represents alkyl and/or alkenyl groups containing 6 to 22 carbon atoms, R⁷ represents hydrogen or alkyl groups containing 1 to 4 carbon atoms, R⁸ represents alkyl groups containing 1 to 4 carbon atoms, q1 is a number of 1 to 6 and Z is an alkali metal and/or alkaline earth metal or ammonium, are used as additional component (e).

12. Use as claimed in at least one of claims 1 to 11, **characterized in that** alkyl amidobetaines corresponding to formula **(V)**: 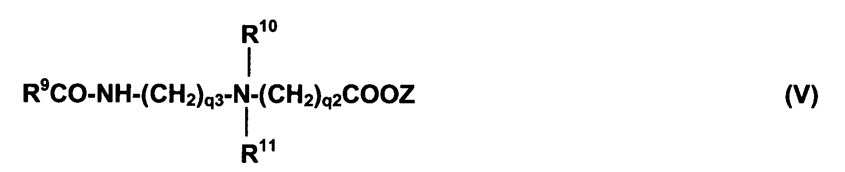 in which R⁹CO is an aliphatic acyl group containing 6 to 22 carbon atoms and 0 or 1 to 3 double bonds, R¹⁰ is hydrogen or represents alkyl groups containing 1 to 4 carbon atoms, R¹¹ represents alkyl groups containing 1 to 4 carbon atoms, q2 is a number of 1 to 6, q3 is a number of 1 to 3 and Z is again an alkali metal and/or alkaline earth metal or ammonium, are used as additional component (e).

13. Use claimed in at least one of claims 1 to 12, **characterized in that**
(a) 1 to 40% by weight anionic surfactants,
(b1) 1 to 15% by weight alkyl and/or alkenyl oligoglycosides and/or
(b2) 1 to 15% by weight amine oxides,
(c) 0.1 to 5% by weight thickeners,
(d) 0.1 to 5% by weight encapsulated active components and optionally
(e) 0 to 7% by weight amphoteric and/or zwitterionic surfactants
are used, with the proviso that the quantities shown add up to 100% by weight with water and optionally other auxiliaries and additives.

## Revendications

1. Utilisation de préparations tensioactives aqueuses de viscosité élevée, contenant :
(a) des tensioactifs anioniques,
(b1) des alkyl- et/ou alcényloligoglycosides et /ou
(b2) des oxydes d'amines,
(c) des agents épaississants,
(d) des substances actives encapsulées ainsi que, le cas échéant,
(e) jusqu'à la moitié de la quantité pondérale de tensioactifs non ioniques (composants b1 et b2) de tensioactifs amphotères et/ou swittérioniques,
pour la préparation de shampooings capillaires.

2. Utilisation selon la revendication 1,
**caractérisée en ce qu'**
on utilise des tensioactifs anioniques choisis dans le groupe formé par les savons, alkylbenzènesulfonates, alcanesulfonates, oléfinesulfonates, éthersulfonates d'alkyle, éthersulfonates de glycérol, alpha-méthylestersulfonates, acides gras sulfonés, sulfates d'alkyle, éthersulfates d'alkyle, éthersulfates de glycérol, éthersulfates d'acides gras, hydroxyéthersulfates mixtes, (éther) sulfates de monoglycérides, (éther)sulfates d'amides d'acides gras, sulfosuccinates de mono- et dialkyle, sulfosuccinamates de mono- et dialkyle, sulfotriglycérides, savons d'amides, acides éthercarboxyliques et leurs sels, iséthionates d'acides gras, sarcosinates d'acides gras, taurures d'acides gras, N-acylaminoacides, sulfates d'alkyloligoglucosides, condensats d'acides gras protéinés et (éther)phosphates d'alkyle.

3. Utilisation selon la revendication 2,
**caractérisée en ce qu'**
on utilise, comme tensioactifs anioniques, des alcanesulfonates, sulfates d'alkyle, éthersulfates d'alkyle et/ou glutamates d'acyle.

4. Utilisation selon au moins une des revendications 1 à 3,
**caractérisée en ce qu'**
on utilise, comme tensioactifs anioniques, des éthersulfates d'alkyle répondant à la formule (I)
**R¹O(CH₂CH₂O)ₙSO₃X** (I)
dans laquelle R¹ représente un radical alkyle ou alcényle linéaire ou ramifié, comportant 6 à 22 atomes de carbone, n représente des nombres de 1 à 10 et X représente un métal alcalin, alcalinoterreux, l'ammonium, un alkylammonium, un alcanolammonium ou le glucammonium.

5. Utilisation selon au moins une des revendications 1 à 4,
**caractérisée en ce qu'**
on utilise des alkyl-et alcènyloligoglycosides répondant à la formule (II),
**R²O-[G]ₚ** (II)
dans laquelle R² représente un radical alkyle et/ou alcényle comportant 4 à 22 atomes de carbone, G représente un radical de sucre comportant 5 ou 6 atomes de carbone et p représente des nombres de 1 à10.

6. Utilisation selon au moins une des revendications 1 à 5,
**caractérisée en ce qu'**
on utilise des oxydes d'amines répondant à la formule (III),
**R³R⁴R⁵-N->O** (III)
dans laquelle R³ représente un radical alkyle ou alcényle linéaire ou ramifié, comportant 6 à 22, de préférence 12 à 18 atomes de carbone, ou un radical benzyle et R⁴ et R⁵ représentent, indépendamment l'un de l'autre, R³ ou des radicaux alkyle linéaires ou ramifiés comportant 1 à 4 atome(s) de carbone.

7. Utilisation selon au moins une des revendications 1 à 6,
**caractérisée en ce qu'**
on utilise des agents épaississants choisis dans le groupe formé par les acides siliciques hydrophiles, polysaccharides, carboxyméthylcelluloses, hydroxyéthyl- et hydroxypropylcelluloses, mono- et diesters de polyéthylèneglycols et d'acides gras de haut poids moléculaire, polyacrylates, polyacrylamides, alcools polyvinyliques, polyvinylpyrrolidones et bentonites.

8. Utilisation selon au moins une des revendications 1 à 7,
**caractérisée en ce qu'**
on utilise des substances actives micro-encapsulées choisies dans le groupe formé par les tensioactifs cationiques, substances actives biogènes, colorants, pigments colorés, huiles parfumées, arômes et leurs mélanges.

9. Utilisation selon la revendication 8,
**caractérisée en ce qu'**
on utilise des substances actives biogènes choisies dans le groupe formé par le squalane, le chitosane, le menthol, le rénitol (vitamine A), la caféïne, les protéines végétales et leurs produits d'hydrolyse, les carotènes et l'huile de jojoba ainsi que leurs mélanges.

10. Utilisation selon au moins une des revendications 1 à 9,
**caractérisée en ce qu'**
on utilise des microcapsules de diamètres moyens situés dans une plage de 0,0001 à 5 mm, constituées d'une membrane enveloppe et d'une matrice contenant les substances actives, que l'on peut obtenir
(a1) en préparant une matrice à partir d'agents gélifiants, de chitosanes et de substances actives,
(a2) en dispersant, le cas échéant, la matrice dans une phase huileuse,
(a3) en traitant la matrice dispersée avec des solutions aqueuses de polymères anioniques et, le cas échéant, en éliminant la phase huileuse,
ou
(b1) en préparant une matrice à partir d'agents gélifiants, de polymères anioniques et de substances actives,
(b2) en dispersant, le cas échéant, la matrice dans une phase huileuse,
(b3) en traitant la matrice dispersée avec des solutions aqueuses de chitosane et en éliminant, le cas échéant, la phase huileuse,
ou
(c1) en traitant des préparations aqueuses de substances actives avec des corps huileux en présence d'émulsionnants pour obtenir des émulsions huile dans l'eau,
(c2) en traitant les émulsions ainsi obtenues avec des solutions aqueuses de polymères anioniques,
(c3) en mettant en contact la matrice ainsi obtenue avec des solutions aqueuses de chitosane, et
(c4) en séparant les produits d'encapsulement ainsi obtenus, de la phase aqueuse.

11. Utilisation selon au moins une des revendications 1 à 10,
**caractérisée en ce qu'**
on utilise comme autre composant (e) des alkylbetaïnes répondant à la formule (IV), 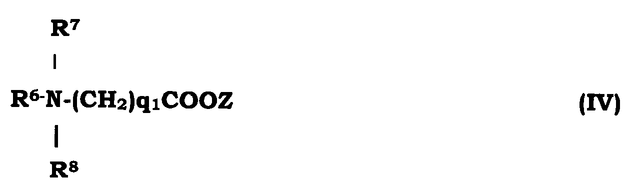 dans laquelle R⁶ représente des radicaux alkyle et /ou alcényle comportant 6 à 22 atomes de carbone, R⁷ représente un atome d'hydrogène ou des radicaux alkyle comportant 1 à 4 atome(s) de carbone, R⁸ représente des radicaux alkyle comportant 1 à 4 atome(s) de carbone, q1 représente des nombres de 1 à 6 et Z représente un métal alcalin et/ou alcalinoterreux ou l'ammonium.

12. Utilisation selon au moins une des revendications 1 à 11,
**caractérisée en ce qu'**
on utilise, comme autre composant (e), des alkylamidobétaïnes répondant à la formule (V) 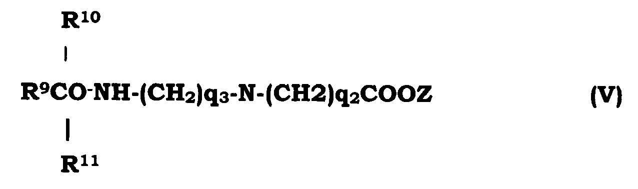 dans laquelle R⁹ CO représente un radical acyle aliphatique comportant 6 à 22 atomes de carbone et 0 ou 1 à 3 double(s) liaison(s), R¹⁰ représente un atome d'hydrogène ou des radicaux alkyle comportant 1 à 4 atome(s) de carbone, R¹¹ représente des radicaux alkyle comportant 1 à 4 atome(s) de carbone, q2 représente des nombres de 1 à 6, q3 représente des nombres de 1 à 3 et Z représente un métal alcalin et/ou alcalinoterreux ou l'ammonium.

13. Utilisation selon au moins une des revendications 1 à 12,
**caractérisée en ce qu'**
on utilise
(a) 1 à 40 % en poids de tensioactifs anioniques,
(b1) 1 à 15 % en poids d'alkyl-et/ou alcényloligoglycosides et/ou
(b2) 1 à 15 % en poids d'oxydes d'amines,
(c) 0,1 à 5 % en poids d'agents épaississants,
(d) 0,1 à 5 % en poids de substances actives encapsulées ainsi que, le cas échéant,
(e) 0 à 7 % en poids de tensioactifs amphotères et/ou zwitterioniques
étant précisé que les indications de quantité se complètent à 100 % avec de l'eau et, le cas échéant, d'autres adjuvants et additifs.
